Europäisches Patentamt

European Patent Office

Office européen des brevets

(11) Veröffentlichungsnummer: **0 244 720**
**B1**

(12) **EUROPÄISCHE PATENTSCHRIFT**

(45) Veröffentlichungstag der Patentschrift: **29.08.90**

(21) Anmeldenummer: **87106053.9**

(22) Anmeldetag: **25.04.87**

(51) Int. Cl.⁵: **A 61 F 2/30, A 61 M 31/00**

(54) **Ausnehmungen zur Einlagerung von medikamentös wirksamen Substanzen in orthopädische oder chirurgische Implantate.**

(30) Priorität: **05.05.86 CH 1834/86**

(43) Veröffentlichungstag der Anmeldung:
**11.11.87 Patentblatt 87/46**

(45) Bekanntmachung des Hinweises auf die
Patenterteilung:
**29.08.90 Patentblatt 90/35**

(84) Benannte Vertragsstaaten:
**AT DE FR GB IT**

(56) Entgegenhaltungen:
**EP-A-0 040 457**
**WO-A-84/02264**
**DE-A-3 000 114**
**US-A-4 186 486**
**US-A-4 199 864**

(73) Patentinhaber: **GEBRÜDER SULZER
AKTIENGESELLSCHAFT**
**Zürcherstrasse 9**
**CH-8401 Winterthur (CH)**

(72) Erfinder: **Buchhorn, Ursula**
**Stegemühlenweg 1**
**D-3400 Göttingen (DE)**
Erfinder: **Willert, H. G., Prof. Dr.-med.**
**Schlegelweg 9**
**D-3400 Göttingen (DE)**

(74) Vertreter: **Sparing Röhl Henseler Patentanwälte**
**European Patent Attorneys**
**Rethelstrasse 123**
**D-4000 Düsseldorf 1 (DE)**

EP 0 244 720 B1

**Beschreibung**

Die Erfindung betrifft ein orthopädisches oder chirurgisches Implantat mit einer Ausnehmung zur Einlagerung von medikamentös wirksamen Substanzen, beispielsweise in Verankerungsteile von zementfrei zu verankernden Gelenkendoprothesen, welche Substanzen in das umgebende Gewebe abgegeben werden sollen.

Es ist bekannt (DE—OS 28 39 040), die Implantat-Oberflächen, die an das Gewebe angrenzen, mit Vertiefungen oder Ausnehmungen zu versehen, in denen medikamentös wirksame Substanzen eingelagert sind. Derartige Substanzen sind z.B. Antibiotika oder andere entzündungshemmende Stoffe, Antikoagulationsmittel oder auch die Bildung von Gewebe stimulierende und fördernde Substanzen. Die Abgabe der therapeutischen Substanz erfolgt dabei durch Lösen in Körperflüssigkeit, die den Transport in das lebende Gewebe übernimmt. In die dadurch entstehenden "hohlen" Ausnehmungen wächst das Gewebe ein. Ein solches Einwachsen von Gewebe ist jedoch nicht in allen Fällen, beispielsweise in operativ schlecht zugänglichen Bereichen einer Implantatverankerung, zulässig und erwünscht. Weiterhin verhindert eingewachsenes Gewebe bei Verankerungen mit einer konischen Klemmung, dass sich ein Implantat, beispielsweise der Schaft einer Gelenkendoprothese, durch Einsinken in den Knochen neu verfestigen kann.

Es ist daher das Ziel der vorliegenden Erfindung, in Implantatteilen für medikamentöse Substanzen Ausnehmungen bereitzustellen, in die nach dem "Verbrauch" dieser Substanz kein Gewebe einwachsen kann. Duese Aufgabe wird mit der vorliegenden Erfindung dadurch gelöst, dass die Ausnhemung an der Implantatoberfläche mit einem porösen Deckel verschlossen ist, dessen Porosität derart ausgebildet ist, dass sie den Durchtritt von Substanzen und von die Substanzen lösenden Flüssigkeiten zulässt, ein Einwachsen von Gewebe in den Deckel und die Ausnehmung jedoch verhindert.

Während Poren mit Porengrössen kleiner 50 μm ein Einwachsen von Knochen zumindest stark behindern, erlauben sie einen ungehinderten Durchtritt von Körperflüssigkeit und Wirksubstanz. Die untere Grenze der Porengrösse ist dabei von der Grösse der Moleküle der zu transportierenden Wirkstoffe abhängig.

Die Konzentration der Wirksubstanz und die Dauer ihrer Abgabe kann durch die Löslichkeit und/oder den Abbau von Speicher substanzen gesteuert werden. Für spezielle Infektionsfälle kann entsprechend einem Antibiogram eine spezifische Wirkstoffkombination zusammengestellt werden. Dabei braucht auf eine Temperaturstabilität der Wirksubstanzen keine Rücksicht genommen zu werden, da kein exotherm polymerisierender Knochenzement verwendet wird. Anzahl, Grösse und Verteilung der Ausnehmungen richten sich unter Berücksichtigung einer ausreichenden Gestaltfestigkeit nach therapeutischen

Gesichtspunkten, beispielsweise nach der ausserhalb eines Implantats benötigen Konzentration und/oder nach dem Wirkungsort und Platzbedarf der Wirksubstanz.

Bei einer vorteilhaften Ausführungsform der Erfindung besteht der Deckel aus elastischem Material und ist in die Ausnehmung unter Vorspannung eingeklemmt; hierbei kann der eingeklemmte elastische Deckel mit Vorteil gegen das Gewebe hin vorgewölbt sein, um einen elastischen Verbund mit dem Knochen zu erreichen.

Bei steg- oder blattartigen Verankerungsteilen, z.B. bei blattartigen Schäffen von Femurkopfprothesen, kann es zweckmässig sein, die Ausnehmungen als Durchgangsbohrungen durch einen Verankerungsteil auszuführen und beidseitig mit einem Deckel zu verschliessen. Weiterhin ist es nützlich, wenn der Deckel mindestens nahezu nahtlos in die Implantatfläche übergeht, da damit ein Einwaschen von Gewebe zwischen Implantat und Deckel verhindert oder zumindest erschwert wird.

Ausehmungen für Wirksubstanzen können sowohl an Verankerungsteilen, wie z.B. Femurschäften oder konvexen Flächen von Hüftgelenkspfannen, als auch an anderen konstruktiven Elementen, wie z.B. im Kragen- oder Halsbereich von Femurkopfprothesen oder Seitenflächen von Tibiaplateaus und/oder Kondylengleitbahnen, angebracht sein. Die Querschnitte der Ausnehmungen können beliebig ausgebildet sein; als fabrikatorisch besonders einfach haben sich jedoch kreisrunde Querschnitte erwiesen.

Als Werkstoffe für die Deckel haben sich besonders poröse Kunststoffe, insbesondere Polyäthylen oder poröse Metalle, z.B. Drahtgeflechte oder Sinterwerkstoffe aus gewebefreundlichen Materialien, wie Titan oder Titanlegierungen, bewährt.

Für eine Fixierung der Deckel in den Ausnehmungen bieten sich verschiedene Möglichkeiten an:

Beispielsqeise kann bei einer als Bohrung ausgeführten Ausnehmung für die Aufnahme des Deckels eine konische Hinterschneidung unter der Implantatoberfläche vorgesehen sein, in die ein Deckel, der vorzugsweise aus elastischem Material besteht, eingeklemmt wird.

Eine andere Möglichkeit ist, eine rechtwinklige Nut in der Bohrung unter der Implantatoberfläche anzubringen, in die der Deckel ebenfalls eingeklemmt wird.

Weiterhin können in Deckel und Bohrung Gewinde eingeschnitten sein.

Schliesslich ist es möglich, die Bohrung zur Aufnahme des Deckels an der Implantataussenfläche durch eine Nut zu verbreitern, in deren Seitenfläche ebenfalls ein Gewinde eingeschnitten wird. Der Deckel wird dann mit seinem Aussendurchmesser an die verbreiterte Nut angepasst und ist mit einem Aussengewinde versehen. Werden die Deckel elastisch ausgeführt, so können sie in diesem Fall zusätzlich durch Verformungen bei Einschrauben klemmend fixiert werden.

Im folgenden wird die Erfindung anhand von Ausführungsbeispielen im Zusammenhang mit der Zeichnung näher erläutert.

Fig. 1 zeigt eine Seitenansicht anterior/posterior eines schematisch dargestellten Geradschaftes einer Femurkopfprothese;

Fig. 2 ist ein Schmitt II—II von Fig. 1;

Fig. 3 ist in vergrössertem Massstab ein Ausschnitt aus Fig. 1;

Fig. 4 ist der Schnitt IV—IV von Fig. 3;

Fig. 5 gibt in einem zweiten Ausführungsbeispiel eine mit Ausnehmungen für medikamentöse Substanzen versehene Endoprothese für eine Hüftgelenkspfanne teilweise im Schnitt wieder;

Fig. 6 ist vergrössert dargestellt ein Detail A aus Fig. 5;

Fig. 7 ist eine weitere Ausführungsform der Erfindung, teilweise im Schnitt.

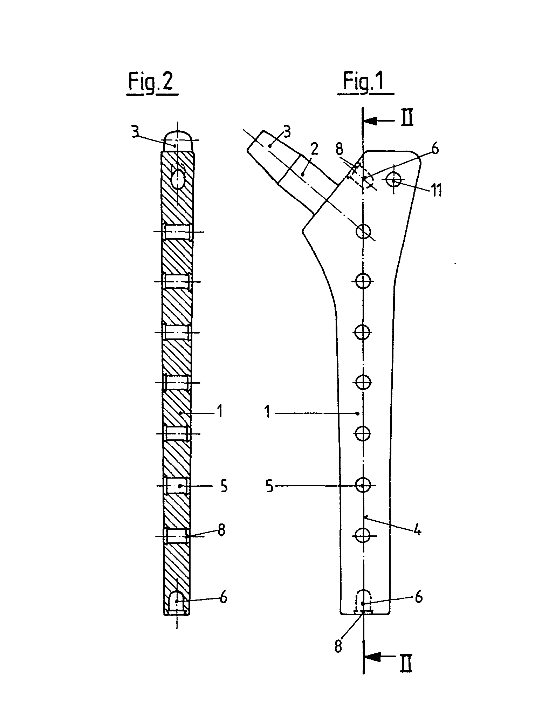

Der Schaft 1 (Fig. 1) einer Femurkopfprothese ist als blattartiger Geradschaft dargestellt, der sich von distal nach proximal konisch erweitert und einen Prothesenhals 2 trägt. Auf diseem sitzt ein Zapfen 3 für die Aufnahme eines nicht gezeigten Gelenkkopfes. Entlang seiner Längsmittelachse 4 besitzt der Schaft 1 eine Vielzahl von Ausnehmungen, die für die Aufnahme von medikamentös wirksamen Substanzen vorgesehen sind. Diese Ausnehmungen sind einesteils Durchgangsbohrungen 5 durch das Schaftblatt und anderenteils Sacklöcher 6 am distalen Ende und in der Ansatzfläche für den Prothesenhals 2.

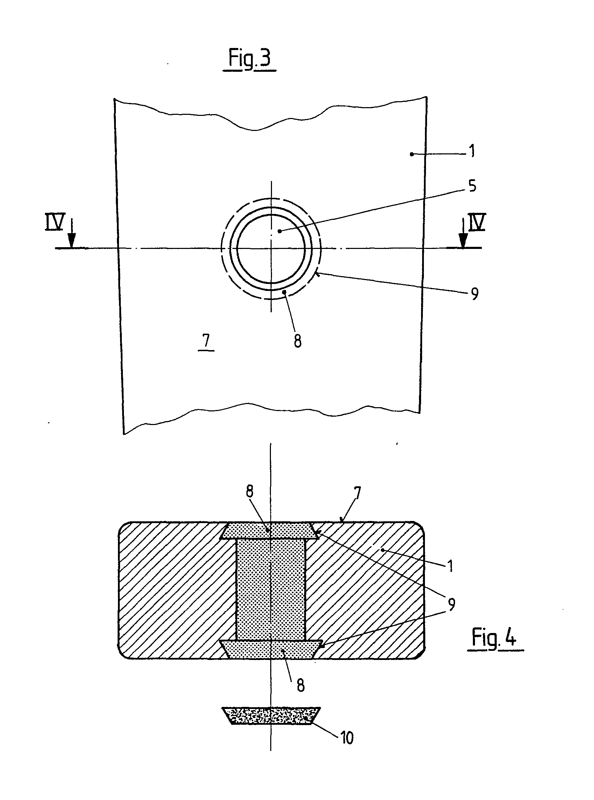

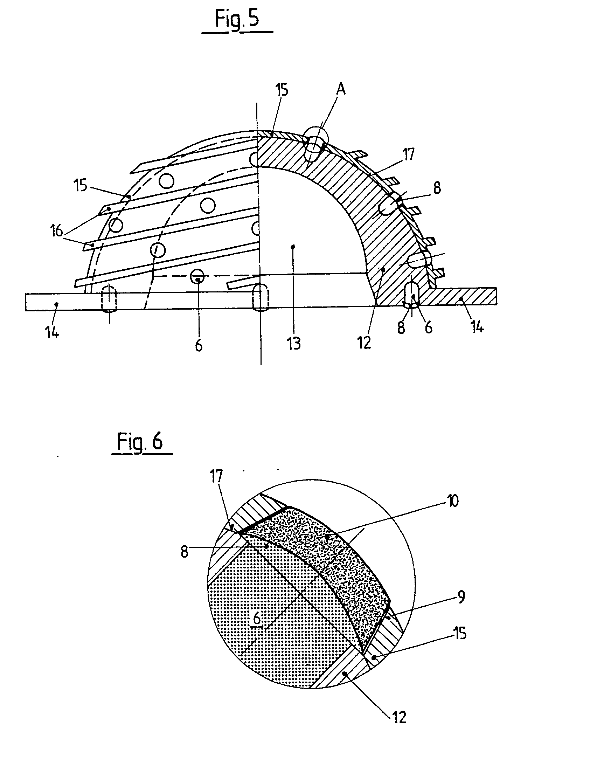

Wie Fig. 3 und 4 zeigen, sind die Durchgangsbohrungen an den beiden Oberfläche 7 der Blattseiten durch eine eingestochene Nut 8, die Hinterschneidungen 9 aufweist, erweitert. Die erweiterten Nuten 8 dienen zur Aufnahme eines porösen Deckels 10, beispielsweise aus Polyäthylen. Der Durchmesser des Deckels 10 ist geringfügig grösser als die eingestochene Nut 8. Die Konizität des Nut 8 unterstützt eine leichte Wölbung des Polyäthylen-Deckels 10 und gewährleistet eine bleibende Spannung, die einen sicheren dauerhaften Halt des Deckels 10 gegen die auftretenden Scherkräfte, beispielsweise während der Implantation, ergibt. Die mit Deckel 10 verschlossene Durchgangsbohrung 5 bildet einen Raum zur Aufnahme einer medikamentös wirksamen Substanz. An Stellen geringer Materialquerschnitte und/oder zum Zwecke einer spezifischen lokalen Anordnung sind Ausnehmungen auch in der Form von Sacklöchern 6 (Fig. 1) vorgesehen; sie weisen die gleichen Nuten 8 für die Deckel 10 wie die Durchgangsbohrungen 5 auf.

Eine weitere Bohrung 11 (Fig. 1) im zum Trochanter major hin sich flügelartig erweiternden proximalen Ende des Schaftes 1 dient als Angriffspunkt für den Ansatz eines Ausziehinstrumentes bei eventuell notwendigen Reoperationen.

Fig. 5 zeigt eine zweiteilige Pfannenprothese für ein Hüftgelenk; der aus Kunststoff gefertigte eigentliche Pfannenkörper 12, der die Pfannenschale 13 für die Aufnahme eines nicht gezeigten Gelenkkopfes enthält, ist auf seiner halbkugelförmigen äusseren Oberfläche 17, die äquatorseitig in einen Rand 14 übergeht, von einer metallenen Aussenschale 15 umschlossen. Für eine Verankerung im Beckenknochen trägt die Aussenschale 15, de beispielsweise aus Titan, einer Titanlegierung oder anderen gewebefreundlichen metallischen Werkstoffen bestehen kann, ein Schraubengewinde 16.

Für eine Aufnahme von medikamentös wirksamen Substanzen sowohl im Rand 14 als auch über die äussere Oberfläche 17 des Pfannenkörpers 12 sind Sacklöcher 6 verteilt. In der freien Oberfläche des Randes 14 bzw. in der Aussenschale 15 sind wiederum Nuten 8 mit Hinterschneidungen 9 vorgesehen.

Während die Ausnehmungen in der äusseren Oberfläche 17 bzw. der Aussenschale 15 das anliegende Gewebe des Implantatlagers medikamentös versorgen, sind die Löcher 6 im Rand des Pfannenkörpers 12—ebenso wie diejenigen in der Ansatzfläche für den Prothesenhals 2 nach Fig. 1 und 2—in den Gelenkinnenraum gerichtet.

In die Hinterschneidungen 9 werden wiederum Deckel 10 (Fig. 6) eingeklemmt. Ihr Durchmesser ist relativ zu demjenigen der Nuten 8 so gewählt, dass der eingespannte Deckel 10 eine deutliche konvexe Wölbung gegen aussen aufweist. Auf diese Weise wird das "Anliegen" des Deckels 10 am Knochen gewährleistet bzw. gefördert.

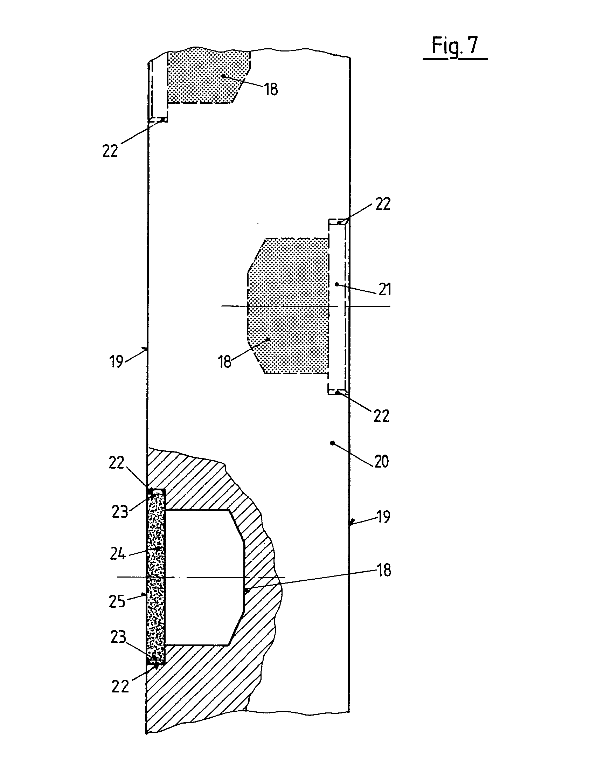

Erlaubt die zu erhaltende Festigkeit des Implantats keine Durchgangsbohrungen wie in Fig. 1 und 2, so besteht die Möglichkeit, mit alternierend versetzt verteilten Sacklöchern 18 (Fig. 7) Ausnehmungen auch in schmalen Implantatelementen unterzubringen; sie sind vorzugsweise ausserhalb der Hauptbelastungsrichtungen angeordnet. Die Sacklöcher 18 sind gegen die Oberfläche 19 des Implantats 20 hin wiederum zu einer Nut 21 erweitert, in deren zylindrischem Mantel ein Gewinde 22 vorgesehen ist. Entsprechend ist der Deckel 24 mit einem Aussengewinde 23 versehen und wird zu seiner Fixierung in die Nut 21 eingeschraubt.

Um an der Grenze zwischen Deckel 24 und Implantatoberfläche 19 eine Einwaschen von Gewebe zu erschweren oder zu verhindern, gehen die äussere Oberfläche 25 des Deckels 24 und die Implantatoberfläche 19 möglichst nahtlos ineinander über.

**Patentansprüche**

1. Orthopädisches oder chirurgisches Implantat mit einer Ausnehmung zur Einlagerung von medikamentös wirksamen Substanzen, beispielsweise in Verankerungsteile von zementfrei zu verankernden Gelenkendoprothesen, welche Substanzen in das umgebende Gewebe abgegeben werden sollen, dadurch gekennzeichnet, dass die Ausnehmung (5, 6; 18) an der Implantatoberfläche (7; 19) mit einem porösen Deckel (10; 24) verschlossen ist, dessen Porosität derart ausgebildet ist, dass sie den Durchtritt von Substanzen und von die Substanzen lösenden Flüssigkeiten zulässt, ein Einwachsen von Gewebe in den Deckel (10; 24) und die Ausnehmung (5, 6; 18) jedoch verhindert.

2. Implantat mit einer Ausnehmung nach Anspruch 1, dadurch gekennzeichnet, das die Porosität des Deckels (10; 24) maximal 50 µm beträgt.

3. Implantat mit einer Ausnehmung nach Anspruch 1, dadurch gekennzeichnet, dass der Deckel (10; 24) aus elastischem Material besteht und in die Ausnehmung (5, 6; 18) unter Vorspannung eingeklemmt ist.

4. Implantat mit einer Ausnehmung nach Anspruch 3, dadurch gekennzeichnet, dass der eingeklemmte elastische Deckel (10) gegen das Gewebe hin vorgewölbt ist.

5. Implantat mit einer Ausnehmung nach Anspruch 1, dadurch gekennzeichnet, dass sie als Durchgangsbohrung (5) durch einen Verankerungsteil (1) ausgeführt ist, die beidseitig von einem Deckel (10) verschlossen ist.

6. Implantat mit einer Ausnehmung nach Anspruch 1, dadurch gekennzeichnet, dass der Deckel (10; 24) mindestens nahezu nahtlos in die Implantatfläche (19) übergeht.

## Revendications

1. Implant orthopédique ou chirurgical présentant un évidement pour le stockage de substances médicamenteuses, par exemple dans des éléments d'ancrage d'endoprothèses d'articulations, à ancrer sans ciment, lesquelles substances doivent être délivrées au tissu environnant, caractérisé en ce que l'évidement (5, 6; 18) est fermé à la surface (7; 19) de l'implant par un couvercle (10; 24) poreux dont la porosité est telle qu'elle autorise le passage de substances et de liquides dissolvant ces substances, mais empêche une croissance du tissu dans le couvercle (10; 24) et dans l'évidement (5, 6; 18).

2. Implant comportant un évidement selon la revendication 1, caractérisé en ce que la porosité du couvercle (10; 24) est de 50 µm au maximum.

3. Implant présentant un évidement selon la revendication 1, caractérisé en ce que le couvercle (10; 24) est réalisé dans un matériau élastique et est serré sous précontrainte dans l'évidement (5, 6; 18).

4. Implant présentant un évidement selon la revendication 3, caractérisé en ce que le couvercle élastique (10) serré est pré-cintré vers le tissu.

5. Implant présentant un évidement selon la revendication 1, caractérisé en ce que celui-ci est un trou débouchant (5) pratiqué dans un élément d'ancrage (1) et fermé des deux côtés par un couvercle (10).

6. Implant présentant un évidement selon la revendication 1, caractérisé en ce que le couvercle (10; 24) se prolonge au moins à peu près sans joint par la surface de l'implant (19).

## Claims

1. An orthopedic or surgical implant formed with a recess for receiving substances which are effective as medicaments, for example, in fixing members of uncemented hip joint replacements, which substances are required to discharge into the surrounding tissue, characterised in that the recess (5, 6; 18) in the implant surface (7; 19) is closed by a porous cover (10, 24) of a porosity such as to permit the passage of substances and of liquids dissolving the same but to inhibit an invasion of tissue into the cover (10; 24) and recess (5, 6; 18).

2. An implant according to Claim 1, characterised in that the porosity of the cover (10; 24) is at most 50 µm.

3. An implant according to Claim 1, characterised in that the cover (10; 24) is made of resilient material and is clamped into the recess (5; 6; 18) with biasing.

4. An implant according to Claim 3, characterised in that the clamped-in resilient cover (10) bulges out towards the tissue.

5. An implant according to Claim 1, characterised in that it is in the form of a continuous bore (5) which extends through a fixing member (1) and which is closed at both ends by a cover (10).

6. An implant according to Claim 1, characterised in that the cover (10; 24) merges at least substantially seamlessly into the implant surface (19).

Fig.2    Fig.1

Fig.3

1

5

IV

IV

9

7

8

8

Fig.4

8

7

1

9

9

8

10

Fig. 5

Fig. 6

Fig. 7